# EUROPÄISCHE PATENTSCHRIFT

(11) **EP 3 672 527 B1**
(45) Veröffentlichungstag und Bekanntmachung des Hinweises auf die Patenterteilung: **06.11.2024**
(21) Anmeldenummer: 18779561.2
(22) Anmeldetag: 06.09.2018
(51) Int. Cl.: A61F 2/24, A61F 2/06, A61F 2/07

(54) **IMPLANTIERBARE KLAPPENPROTHESE**
IMPLANTABLE VALVE PROSTHESIS
PROTHÈSE VALVULAIRE IMPLANTABLE

(30) Priorität: 13.09.2017 DE 102017121143
(43) Veröffentlichungstag der Anmeldung: 01.07.2020
(73) Patentinhaber: Universitätsklinikum Hamburg-Eppendorf, 20246 Hamburg (DE)
(72) Erfinder: YILDIRIM, Yalin, 22529 Hamburg (DE); PECHA, Simon, 22529 Hamburg (DE); REICHENSPURNER, Hermann, 20251 Hamburg (DE)
(74) Vertreter: Stüven, Ralf
(86) Internationale Anmeldenummer: PCT/DE2018/200084
(87) Internationale Veröffentlichungsnummer: WO 2019/052610

(56) Entgegenhaltungen:
- WO-A1-2006/083763
- WO-A1-2009/153768
- WO-A1-2014/165010
- WO-A2-2008/046092
- FR-A1- 2 788 217
- US-A- 5 855 597
- US-A1- 2004 102 855
- US-A1- 2006 111 773
- US-A1- 2013 289 711
- US-A1- 2013 289 740
- US-B2- 7 771 467

## Beschreibung

Die Erfindung betrifft eine implantierbare Klappenprothese, insbesondere zur Verhinderung von Blutrückfluss aus einem Herzvorhof in eine in den Herzvorhof mündende Vene.

Die Mitralklappe dient dazu, den Rückfluss von Blut aus der linken Herzkammer in den linken Herzvorhof während der Systole zu verhindern. Die Mitralklappeninsuffizienz hat allerdings eine hohe Prävalenz. Klinisches Hauptsymptom einer schweren Mitralklappeninsuffizienz ist die Dyspnoe, welche durch einen Blutrückstau in die Lunge verursacht wird. Neben der chirurgischen Mitralklappenrekonstruktion oder dem Ersatz stehen interventionelle Verfahren zur Verfügung. Als Ersatz der Mitralklappe sind beispielsweise Mitralklappenprothesen im Stand der Technik beschrieben worden (s. z.B. US 5258023 A, US 7641686 B2, Vaquerizo et al. 2015, Percutaneous Transcatheter Mitral Valve Replacement: Patient-specific Three-dimensional Computer-based Heart Model and Prototyping, Rev. Esp.Cardiol. 68, 1165-1173, doi: 10.1016/j.rec.2015.08.005; Jamieson et al. 2009, Mitroflow aortic pericardial bioprosthesis - clinical performance, Eur J Cardiothorac Surg 36, 818-824, doi: 10.1016/j.ejcts.2009.05.020).

Die Trikuspidalklappe hat die Funktion, den Rückfluss von Blut aus der rechten Herzkammer in den rechten Herzvorhof während der Systole zu verhindern. Die Trikuspidalklappeninsuffizienz stellt bei Patienten mit einer eingeschränkten rechtsventrikulären Herzfunktion ein häufiges klinisches Problem dar. Neben der chirurgischen Trikuspidalklappenrekonstruktion oder dem Ersatz stehen bisher keine klinisch etablierten interventionellen Verfahren zur Verfügung. Die klinischen Hauptsymptome der schweren Trikuspidalinsuffizienz sind ein Blutrückstau in Leber und Niere, Aszites und Ödeme. Über längere Zeit entwickeln sich dadurch Leber- und Nierenfunktionsstörungen.

Die bekannten Prothesen und Maßnahmen sind aber nicht bei allen Patienten erfolgversprechend, da aufgrund verschiedener anatomischer sowie klappenmorphologischer Umstände wie z.B. einer starken Dilatation des Mitral- oder Trikuspidalrings oder anderen anatomischen Gegebenheiten interventionelle Verfahren zur Reparatur der Mitralklappe oder Trikuspidalklappe an Ihre Grenzen stoßen. WO 2014/165010 beschreibt ein röhrenförmiges geflochtenes Netzgerüst zur Herstellung von Herzklappen mit einer Hülle, die die Klappensegel ausbildet.

Aufgabe der vorliegenden Erfindung ist es daher, eine Alternative zu den bisher verfügbaren Mitteln zur Behandlung einer Mitralklappen- oder Trikuspidalklappeninsuffizienz zur Verfügung zu stellen, welche insbesondere unabhängig von der Anatomie und Morphologie der Mitral- und Trikuspidalklappen eingesetzt werden kann.

Gelöst wird die Aufgabe durch eine implantierbare Klappenprothese, insbesondere zur Verhinderung von Blutrückfluss aus einem Herzvorhof in eine in den Vorhof mündende Vene, umfassend
- einen allgemein röhrenförmigen Stent, der verzweigt oder unverzweigt sein kann und mindestens einen ersten Endabschnitt mit einer ersten Endöffnung und einen zweiten Endabschnitt mit einer zweiten Endöffnung aufweist, und
- einen flexiblen Schlauch, der verzweigt oder unverzweigt sein kann und mindestens einen ersten Endabschnitt mit einer ersten Endöffnung und einen zweiten Endabschnitt mit einer durch Kollabieren des Schlauches verschließbaren zweiten Endöffnung aufweist, der zumindest in einem Teilbereich des zweiten Endabschnitts des Stents auf dessen äußerer Umfangsfläche so angeordnet ist, dass der Schlauch mit seinem zweiten Endabschnitt und der zweiten Endöffnung über die zweite Endöffnung des Stents hinaussteht, wobei die zweite Endöffnung des Schlauches im drucklosen Zustand offen ist.

Die erfindungsgemäße Klappenprothese ist interventionell implantierbar und besonders gut dafür geeignet, einen Blutrückfluss aus dem linken Vorhof durch die Pulmonalvenen zur Lunge oder einen Blutrückfluss aus dem rechten Vorhof in die Hohlvenen zu verhindern oder zu minimieren, und damit in einer symptomatischen Therapie der Mitralklappeninsuffizienz oder Trikuspidalklappeninsuffizienz eingesetzt zu werden.

Die Klappenprothese ist so ausgestaltet, dass sie interventionell mit einem Ende in einer Pulmonal- oder Hohlvene verankert werden kann, während sie mit einem anderen Ende frei in den zugehörigen Vorhof ragt. Die Klappenprothese ist dabei so ausgestaltet, dass die Klappe im Neutralzustand, d.h. ohne dass das in den Vorhof ragende Schlauchende mit einem äußeren Über- oder inneren Unterdruck beaufschlagt ist, offen ist, während die Klappe bei Druck von außen oder Unterdruck von innen durch Kollabieren des Schlauchendes geschlossen ist. Eine Beaufschlagung des Schlauchendes mit einem äußeren Über- bzw. inneren Unterdruck kann beispielsweise Folge eines Rückflusses von Blut aus dem kontrahierenden linken Ventrikel in den linken Vorhof durch eine unzureichend dichtende Mitralklappe oder eines Rückflusses von Blut aus dem kontrahierenden rechten Ventrikel in den rechten Vorhof durch eine unzureichend dichtende Trikuspidalklappe sein.

Aufgrund dieses Klappendesigns kommt es beim physiologischen Fluss aus den Pulmonalvenen in Richtung linkem Vorhof oder aus den Hohlvenen in Richtung rechtem Vorhof zu keinem oder einem nur unwesentlich erhöhten Flusswiderstand. Im Vergleich zu bekannten Herzklappen, welche in Normalposition geschlossen sind und erst durch Überwindung eines Widerstands geöffnet werden, ist der Flusswiderstand bei der erfindungsgemäßen Klappe nahezu vernachlässigbar. Bei einem unphysiologischen Rückfluss aus dem Vorhof in Richtung der der in den Vorhof mündenden Venen kommt es durch einen dadurch verursachten Unterdruck im Inneren des in den Vorhof ragenden Schlauchendes bzw. einen Überdruck im Vorhof zu einem Kollaps der Schlauchklappe, und somit zu einem kompetenten Klappenschluss. Ein Rückstrom in die Lunge und die damit verbundene Dyspnoe als Hauptsymptom der Mitralinsuffizienz oder ein Rückstrom in die Hohlvenen und somit ein konsekutiver Blutrückstau in Leber und Nieren wird somit verhindert, ohne dass die Klappe ein wesentliches Flusshindernis für den physiologischen Blutfluss darstellt. Somit kann Patienten, bei denen eine chirurgische Therapie zu risikoreich und ein bisher gängiges interventionelles Verfahren aus anatomischen Gründen nicht möglich ist, eine symptomatische Therapie der Mitral- oder Trikuspidalinsuffizienz angeboten werden. Dies ist völlig unabhängig von der Pathologie und den anatomischen Gegebenheiten der Mitral- oder Trikuspidalklappe.

Der Ausdruck "zur Verhinderung von Blutrückfluss aus einem Herzvorhof in eine in den Herzvorhof mündende Vene" in Bezug auf die erfindungsgemäße implantierbare Klappenprothese bezieht sich auf die Eignung der Klappenprothese zur Verhinderung von Blutrückfluss aus dem linken Herzvorhof in eine Pulmonalvene oder aus dem rechten Herzvorhof in eine Hohlvene.

Der Begriff "interventionell implantierbar" in Bezug auf die erfindungsgemäße Klappenprothese bedeutet, dass die Klappenprothese minimal-invasiv mittels eines Gefäßkatheters implantiert werden kann.

Unter einem "Stent" wird eine implantierbare Stütz- oder Versteifungsvorrichtung zum Öffnen oder Offenhalten von Gefäßen oder Hohlorganen verstanden. Sie bestehen aus biokompatiblem Material, beispielsweise Metall oder Kunststoff. Beispielsweise kann es sich um eine allgemein röhrenförmige Struktur aus einem Metall- und/oder Kunststofffaser-Gitter oder -Geflecht handeln.

Unter einem "allgemein röhrenförmigen Stent" wird ein Stent verstanden, der allgemein tubulär ausgebildet ist. Beispielsweise kann der Stent als allgemein hohlzylindrischer Schlauch ausgebildet sein, wobei der Querschnitt rund oder elliptisch sein kann. Unter einem "verzweigten Stent" wird ein Stent verstanden, der mindestens eine Gabelung aufweist. Beispielsweise kann der Stent eine Y-Form aufweisen, so dass sich eine Röhre an einer Gabelung in zwei Röhren aufteilt. Verzweigte, beispielsweise Y-förmige Stents sind grundsätzlich bereits bekannt und beispielsweise in der EP 0830109 B1 beschrieben.

Wenn hier von einer "Stentöffnung", einer "Endöffnung eines Stents" oder einem "offenen Stentende" gesprochen wird, bezieht sich dies selbstverständlich nicht auf eine Öffnung zwischen Drähten des die Stentwand oder Stentmantels bildenden Drahtgeflechts, sondern auf eine Öffnung, die am Ende eines Stentabschnitts angeordnet und von der Stentwand umgeben ist.

Unter einem "flexiblen Schlauch" wird hier ein Schlauch verstanden, der nicht starr ausgebildet ist, sondern leicht biegsam ist. Insbesondere wird hier unter diesem Begriff ein Schlauch verstanden, der kollabierbar ist, d.h. unter Druckverhältnissen, wie sie unter physiologischen Bedingungen im linken Herzvorhof während der Systole, insbesondere am Ende der Anspannungsphase oder zu Beginn und während der Auswurfphase (Austreibungsphase) der Ventrikelsystole auftreten, in sich zusammenfällt oder zusammengedrückt wird, so dass der Schlauch unter diesen Bedingungen im Wesentlichen geschlossen ist. Der Begriff "physiologisch" schließt hier auch Bedingungen ein, wie sie unter krankhaften Verhältnissen, beispielsweise im Falle einer Mitralklappeninsuffizienz oder Trikuspidalklappeninsuffizienz im menschlichen oder tierischen Körper auftreten können. Unter einem "verzweigten Schlauch" wird hier ein sich in getrennte schlauchförmige Abschnitte aufteilender oder gabelnder Schlauch, beispielsweise Y-förmiger Schlauch verstanden.

Der Begriff "Kollabieren" in Bezug auf einen Endabschnitt des flexiblen Schlauch bezeichnet das Zusammenfallen, Einstülpen, Invaginieren oder Zusammengedrücktwerden des Schlauches in diesem Abschnitt, so dass die Schlauchwand zum Schlauchinneren gezogen oder gedrückt wird und Bereiche der Schlauchinnenwand sich aneinanderlegen und den Schlauch reversibel verschließen. Der Ausdruck, wonach die zweite Endöffnung des Schlauches durch dessen Kollabieren verschließbar ist, bedeutet, dass ein Hindurchfließen von Blut von der zweiten Endöffnung des Schlauches zur ersten Endöffnung des Schlauches durch Verengung des Schlauchquerschnitts in dem Schlauchabschnitt zwischen erster und zweiter Endöffnung im Wesentlichen verhinderbar ist. Das kann eine Querschnittsverengung der zweiten Endöffnung selbst mit einschließen, muss dies jedoch insbesondere bei unten näher beschriebenen Ausführungsformen, bei denen die Endöffnung ein Verstärkungselement aufweist, nicht notwendig.

Der Ausdruck, wonach "die zweite Endöffnung des Schlauches im drucklosen Zustand offen ist" bedeutet, dass sich der zweite Endabschnitt des Schlauches unter Normalbedingungen ohne Druckdifferenz zwischen Schlauchinnerem und Schlauchäußerem nicht in einem kollabierten Zustand befindet und die zweite Endöffnung offen und für Flüssigkeit durchlässig ist. Ein druckloser Zustand, d.h. ein Zustand ohne Druckdifferenz zwischen Innen- und Außenseite des Schlauches, wird hier gegebenenfalls auch als "Neutralzustand" bezeichnet.

Unter dem Begriff "Mitralklappeninsuffizienz" (auch "Mitralinsuffizienz", MI) wird ein Herzklappenfehler verstanden, bei dem die Mitralklappe (auch Bikuspidalklappe) des Herzens, die zwischen dem linken Vorhof (Atrium) und der linken Kammer (Ventrikel) des Herzen angeordnet ist und im funktionsfähigen Zustand einen systolischen Rückfluss von Blut aus der linken Kammer in den linken Vorhof verhindert, in ihrer Funktion beeinträchtigt, d.h. schlussunfähig oder undicht ist, so dass es während der Auswurfphase der Ventrikelsystole zu einem Rückfluss von Blut aus der linken Herzkammer in den linken Vorhof kommt.

Unter dem Begriff "Trikuspidalklappeninsuffizienz" (auch "Trikuspidalinsuffizienz", TI) wird ein Herzklappenfehler verstanden, bei dem die Trikuspidalklappe des Herzens, die zwischen dem rechten Vorhof (Atrium) und der rechten Kammer (Ventrikel) des Herzen angeordnet ist und im funktionsfähigen Zustand einen systolischen Rückfluss von Blut aus der rechten Kammer in den rechten Vorhof verhindert, in ihrer Funktion beeinträchtigt, d.h. schlussunfähig oder undicht ist, so dass es während der Auswurfphase der Ventrikelsystole zu einem Rückfluss von Blut aus der rechten Herzkammer in den rechten Vorhof kommt.

Der Begriff "Pulmonalvenen" oder "Lungenvenen" bezieht sich auf die venae pulmonales, d.h. Blutgefäße, die sauerstoffreiches Blut aus der Lunge zum linken Vorhof des Herzens transportieren. Beim Menschen sind in der Regel vier Pulmonalvenen (untere und obere rechte Pulmonalvene sowie untere und obere linke Pulmonalvene) vorhanden, die getrennt in den linken Vorhof münden.

Der Begriff "Hohlvene" bezieht sich auf die venae cavae, d.h. Blutgefäße, die venöses Blut aus dem Körper zum rechten Herzvorhof transportieren. Der Mensch verfügt in der Regel über zwei Hohlvenen, die obere Hohlvene (Vena cava superior) und die untere Hohlvene (Vena cava inferior)
Die erfindungsgemäße Klappenprothese ist so ausgestaltet, dass das über den Stent hinausstehende Schlauchende im implantierten Zustand kollabiert und dadurch verschlossen wird, wenn sich während der Systole der Druck im Atrium erhöht. Das Offenhalten des Schlauches während der außersystolischen Phasen des Herzzyklus, insbesondere während der Füllungsphase (Diastole) kann entweder durch entsprechende Wahl des Schlauchmaterials und/oder der Wanddicke und/oder durch geeignete Stützelemente bewirkt werden. Der Schlauch weist eine ausreichende Flexibilität/Elastizität auf, um sich durch den passiven Fluss aus der Lunge in das Atrium zu Beginn der Diastole zu öffnen. Wenn sich der Druck im Atrium zu Beginn der Systole erhöht, wird die Klappe geschlossen, indem der Schlauch unter Verschluss der Endöffnung kollabiert.

Die Implantation des Stents kann auf die im Stand der Technik bekannte Weise erfolgen, beispielsweise mittels eines Ballonkatheters. Der zunächst zusammengefaltete Stent wird dabei mittels des Katheters an die gewünschte Stelle, beispielsweise eine Pulmonalvenenmündung oder Hohlvenenmündung in den zugehörigen Vorhof, gebracht und dort aufgedehnt und verankert. Alternativ kann die Klappe aus einem selbstexpandierenden Material bestehen (z.B. einer Nickel-Titan-Legierung, Nitinol), welches sich nach Freisetzen des Stents und in Kontakt mit dem Blutstrom zur vorhergesehenen Größe expandiert. Die Prinzipien der selbstexpandierbaren Klappe sind dem Fachmann bekannt.

In einer bevorzugten Ausführungsform der erfindungsgemäßen Klappenprothese umfasst diese mindestens ein am Stent befestigtes oder mit dem Stent integral ausgebildetes draht- oder bandförmiges Stabilisierungselement, wobei das Stabilisierungselement über die zweite Endöffnung des Stents hinaussteht und den über die zweite Endöffnung des Stents hinausstehenden Teil des Schlauchs so von innen stützt, dass die zweite Endöffnung des Schlauches in drucklosem Zustand offen ist und unter Druckbeaufschlagung nicht in die zweite Endöffnung des Stents gelangen kann. Bei dieser Ausführungsform erstreckt sich ein draht- oder bandförmiges Stabilisierungselement vom Stentende bzw. über das Stentende hinaus in Richtung Schlauchende und stützt den Schlauch von innen, so dass ein Kollabieren, z.B. das Einstülpen des überstehenden Schlauchteils in das Stentinnere, im Neutralzustand verhindert wird. Darüber hinaus verhindert das draht- oder bandförmige Stabilisierungselement auch ein Invaginieren des Schlauchendes in den Stent bei einem äußeren Überdruck oder inneren Unterdruck, insbesondere beim Klappenschluss. Damit wird vermieden, dass der über die zweite Endöffnung des Stents hinausstehende Teil des Schlauchs in das Innere des Stents zurückgedrückt oder -gesogen wird. Das Stabilisierungselement erstreckt sich vorzugsweise bis zum Schlauchende.

Besonders bevorzugt sind mindestens zwei einander gegenüberliegende den Schlauch von innen stützende draht- oder bandförmige Stabilisierungselemente vorhanden. Bei dieser Ausführungsform ist ein Kollabieren des Schlauches im Wesentlichen nur durch Einwärtsbewegung der zwischen den Stabilisierungselementen liegenden Wandbereichen des Schlauches möglich. Es hat sich gezeigt, dass diese Ausführungsform besonders zuverlässig funktioniert und ein ungewolltes Kollabieren des schlauchförmigen Klappenanteils verhindert.

Wie oben beschrieben, bewirkt das mindestens eine draht- oder bandförmige Stabilisierungselement auch, dass eine Invagination des Schlauches zurück in den Stent, z.B. in Unterdrucksituationen, verhindert ist. Alternativ oder auch zusätzlich kann hierfür aber auch vorgesehen sein, dass der Schlauch sich zu seiner zweiten Endöffnung hin im Querschnitt erweitert und/oder in seinem zweiten Endabschnitt ein außen am Schlauch befestigtes ringförmiges Verstärkungselement aufweist. Der Innenquerschnitt des ringförmigen Verstärkungselements entspricht bevorzugt mindestens dem Innenquerschnitt der zweiten Endöffnung des Stents, so dass ein Eindringen des Schlauches mit seiner zweiten Endöffnung in den Stent hinein nicht möglich ist. Durch die Erweiterung zur zweiten Endöffnung hin wird aufgrund der Vergrößerung des Schlauchquerschnitts auch der Flusswiderstand weiter verringert. Das ringförmige Verstärkungselement versteift den Schlauch in seinem zweiten Endabschnitt, beispielsweise im Bereich seiner zweiten Endöffnung und sorgt dafür, dass der Schlauch nicht in den Stent zurückgezogen werden kann. Bei dieser Ausführungsform wird die Länge des Schlauchabschnitts zwischen dem zweiten Stentende und dem ringförmigen Verstärkungselement so gewählt, dass sich der Schlauch in diesem Abschnitt vollständig verschließen kann. Das ringförmige Verstärkungselement kann sowohl bei Ausführungsformen mit Querschnittserweiterung des Schlauches zur zweiten Endöffnung hin als auch bei Ausführungsformen ohne eine solche Querschnittserweiterung angeordnet sein. Im Falle einer Ausführungsformen mit Querschnittserweiterung ist der Innenquerschnitt des ringförmigen Verstärkungselements größer als der Innenquerschnitt der zweiten Endöffnung des Stents.

Die erfindungsgemäße implantierbare Klappenprothese ist vorzugsweise so ausgestaltet, dass sich bei vollständigem Schluss der Klappenprothese eine Koaptationslänge L von mindestens 1 cm, bevorzugt mindestens 1,2 cm, 1,3 cm oder 1,5 cm ergibt. Unter der Koaptationslänge wird die Länge des Schlauchbereichs in Längsrichtung der Klappenprothese, d.h. in Flussrichtung des durch die Klappenprothese hindurchfließenden Blutes, verstanden, über die sich die gegenüberliegenden Schlauchanteile beim Schluss der Klappenprothese maximal überlappen. Die gewünschte Koaptationslänge kann beispielsweise durch eine für das jeweilige Klappendesign geeignet gewählte Schlauchlänge angepasst werden.

In einer Ausführungsform der Erfindung, die besonders gut zur Verhinderung von Blutrückfluss aus dem rechten Herzvorhof in eine Hohlvene geeignet ist, sind zwei implantierbare Klappenprothesen, wie sie oben beschrieben sind, miteinander zu einer Klappenprothese verbunden. In dieser Ausführungsform umfasst die erfindungsgemäße implantierbare Klappenprothese eine erste und eine zweite implantierbare Klappenprothese, wie sie oben beschrieben ist, wobei
- die erste und zweite implantierbare Klappenprothese so einander gegenüberliegend angeordnet sind, dass sie mit ihren zweiten Schlauchendöffnungen aufeinander zugerichtet sind,
- die erste und zweite implantierbare Klappenprothese mit ihren zweiten Schlauchendöffnungen voneinander beabstandet sind,
- die erste und zweite implantierbare Klappenprothese jeweils ein am Stent befestigtes oder mit dem Stent integral ausgebildetes draht- oder bandförmiges Stabilisierungselement aufweisen, das sich über die Schlauchendöffnungen der ersten und zweiten implantierbaren Klappenprothese hinaus erstreckt, und
- die erste und zweite implantierbare Klappenprothese durch das mindestens eine draht- oder bandförmige Stabilisierungselement miteinander verbunden sind.

Bei dieser Ausführungsform sind zwei implantierbare Klappenprothesen über das mindestens eine draht- oder bandförmige Stabilisierungselement miteinander gekoppelt, so dass sie mit ihren zweiten Schlauchendöffnungen aufeinander zugerichtet sind. Die örtliche Stabilisierung der Klappenprothese ist bei dieser Ausführungsform erhöht. Durch die Beabstandung der Schlauchendöffnungen voneinander kann Blut im offenen Zustand der Klappe, d.h. während der Diastole, zwischen den zweiten Schlauchendöffnungen in das Atrium gelangen. Diese Ausführungsform der implantierbaren Klappenprothese kann mit dem ersten Endabschnitt der ersten Klappenprothese in der oberen und mit dem ersten Endabschnitt der zweiten Klappenprothese in der unteren Hohlvene implantiert werden. Die Klappenprothese weist eine ausreichende Flexibilität auf, um sich an verschiedene anatomische Gegebenheiten anzupassen. Die Schlauchlängen und der Abstand zueinander können so angepasst werden, dass ein zuverlässiger Klappenschluss während der Systole erfolgt. Auch bei dieser Ausführungsform können beispielsweise zwei draht- oder bandförmige Stabilisierungselemente vorgesehen sein, die einander gegenüberliegen oder auch lediglich in Umfangsrichtung versetzt zueinander angeordnet sein können.

In einer besonders bevorzugten Ausführungsform der erfindungsgemäßen implantierbaren Klappenprothese sind der Stent und der Schlauch unverzweigt und allgemein hohlzylindrisch ausgebildet. Diese Ausführungsform ist besonders einfach herzustellen. Der Stent ist allgemein hohlzylindrisch ausgebildet und kann mit seinem ersten offenen Ende beispielsweise in eine Pulmonal- oder Hohlvene eingebracht und dort verankert werden. Auf dem Abschnitt mit dem zweiten offenen Ende kann der ebenfalls allgemein hohlzylindrisch ausgebildete Schlauch aufgebracht werden, der wiederum mit seinem zweiten Ende über das zweite Stentende hinaussteht und in das Atrium ragt.

In einer alternativen Ausführungsform der erfindungsgemäßen implantierbaren Klappenprothese sind der Stent und/oder der Schlauch verzweigt und allgemein Y-förmig ausgebildet. Bei einer Ausführungsform sind der Stent und der Schlauch Y-förmig ausgebildet. Diese Ausführungsform kann beispielsweise mit zwei ihrer Stentenden in den atrialen Mündungen der oberen und unteren linken, in der oberen und unteren rechten Pulmonalvene oder der oberen und unteren Hohlvene angeordnet werden, während das dritte Stentende das überstehende Schlauchende aufweist. Auf diese Weise können zwei Pulmonalvenen oder die Hohlvenen mit nur einer Klappenprothese ausgestattet werden. Der Querschnitt des das überstehende Schlauchende tragenden Stentabschnitts kann zusammen mit dem entsprechenden Schlauchabschnitt in geeigneter Weise erweitert sein, um eine Flussbegrenzung des durchströmenden Blutes zu vermeiden. In einer weiteren Ausführungsform ist nur der Schlauch Y-förmig ausgebildet, während zwei röhrenförmige Stents in zwei der Schlauchenden eingebracht sind.

Der Schlauch kann aus einem geeigneten biokompatiblen Material, beispielsweise einem Kunststoffmaterial, oder aus Körpergewebe, vorzugsweise aus menschlichem oder tierischem Körpergewebe, bestehen. Geeignete Schlauchmaterialien sind dem Fachmann bekannt.

Der Stent besteht aus einem geeigneten biokompatiblen Material, beispielsweise einem geeigneten Metall, z.B. Titanstahl oder Nitinol, oder Kunststoff, z.B. Polytetrafluorethylen (PTFE) oder Polyetheretherketon (PEEK). Geeignete Stent-Materialien sind dem Fachmann bekannt.

Der Schlauch kann auf der gesamten Oberfläche des Stents angeordnet sein, d.h. der Stent kann im Bereich seiner gesamten äußeren Umfangsfläche von dem Schlauch überzogen sein, so dass der Schlauch auch in dem Bereich vorhanden ist, mit dem der Stent in einer Vene verankert wird. Der Stent kann aber auch in den Bereichen, die zu dessen Verankerung in einer Vene vorgesehen sind, vom Schlauch freigehalten sein, so dass der implantierte Stent unmittelbar mit der Innenwand einer Vene Kontakt hat. Letzteres ist von Vorteil, um eine möglichst geringe Verringerung des Gefäßinnenquerschnitts und damit eine möglichst geringe Flussbehinderung zu erreichen. Der Schlauch kann in geeigneter Weise auf dem Stent befestigt, beispielsweise festgenäht oder geklebt, sein.

Die implantierbare Klappenprothese gemäß der Erfindung kann eine oder mehrere Befestigungselemente, z.B. aus Draht, aufweisen, mittels derer die Klappenprothese beispielsweise mit chirurgischem Nahtmaterial zusätzlich im Körper verankert werden kann. Beispielsweise kann ein drahtförmiges Befestigungselement über eine Implantationsvene (z.B. Vena subclavia) ausgeführt und mit chirurgischem Nahtmaterial fixiert werden. Auf diese Weise kann die implantierbare Klappenprothese beispielsweise extravasal befestigt werden.

Die Erfindung wird im Folgenden anhand der angehängten Figuren sowie anhand eines Ausführungsbeispiels rein zu Veranschaulichungszwecken näher erläutert.
Figur 1. Zwei Ausführungsformen (A, B) der erfindungsgemäßen Klappenprothese in seitlichen Ansichten (oben) und Schnittansichten (unten), bei denen der Stent vollständig (A) oder nur teilweise (B) vom Schlauch überzogen ist.
Figur 2. Vereinfachte räumliche Ansichten der in den Figuren 1A und 1B dargestellten Ausführungsformen der erfindungsgemäßen Klappenprothese.
Figur 3A-C. Ansichten weiterer Ausführungsformen der erfindungsgemäßen Klappenprothese.
Figur 4 Stark schematisierte Darstellung eines linken Herzvorhofs mit in die Mündungen der Pulmonalvenen eingesetzten bevorzugten Ausführungsformen der erfindungsgemäßen Klappenprothese.
Figuren 5, 6. Zwei weitere Ausführungsformen (A, B) der erfindungsgemäßen Klappenprothese in seitlichen Ansichten (oben) und Schnittansichten (unten).
Figur 7. Ansichten zweier Ausführungsformen (A, B) der erfindungsgemäßen Klappenprothese, die besonders zur Implantation in die Hohlvenen geeignet ist.
Figur 8. Räumliche Ansichten der in Figur 7 dargestellten Ausführungsformen der der erfindungsgemäßen Klappenprothese.
Figur 9. Vereinfachte Schnittdarstellung durch ein menschliches Herz mit einer in die Hohlvenen implantierten Ausführungform der erfindungsgemäßen Klappenprothese gemäß den Figuren 7 und 8.
Figur 10. Schnittansicht einer Ausführungsform einer erfindungsgemäßen Klappenprothese in geschlossenem Zustand.
Figur 11. Echokardiographie eines Schweineherzens mit linksatrial implantierter erfindungsgemäßer Klappenprothese. LA = Linkes Atrium, LAK = Linksatriale Klappenprothese.

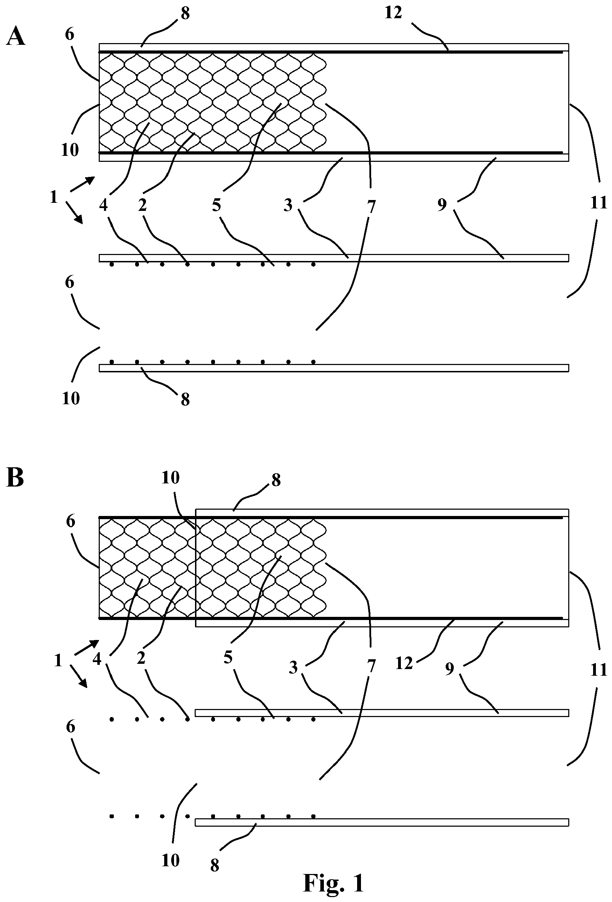

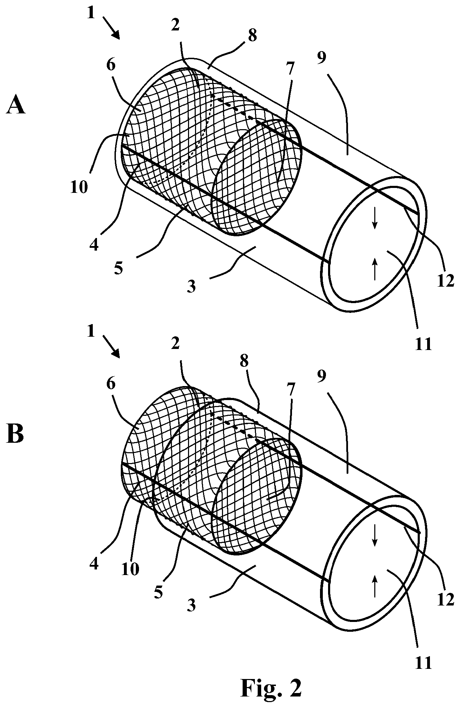

Figur 1 zeigt zwei bevorzugte Ausführungsformen einer erfindungsgemäßen Klappenprothese 1. Im oberen Teil der Figuren ist jeweils eine Seitenansicht dargestellt, im unteren Teil eine Schnittansicht. Die Schnittansicht zeigt einen Schnitt, der nicht in Höhe der bei dieser Ausführungsform gegenüberliegenden (s. auch Fig. 2) draht- oder bandförmigen Stabilisierungselemente 12 liegt. Die in den Figur 1A, B dargestellten Ausführungsformen der erfindungsgemäßen Klappenprothese 1 sind allgemein hohlzylinderförmig ausgestaltet. Ein allgemein hohlzylinderförmig ausgebildeter Stent 2, der aus einem Titanstahlgeflecht gebildet sein kann, weist einen ersten Endabschnitt 4 mit einer ersten Endöffnung 6 und einen zweiten Endabschnitt 5 mit einer der ersten Endöffnung 6 gegenüberliegenden zweiten Endöffnung 7 auf. Ein ebenfalls hohlzylindrischer flexibler Schlauch 3, der ein Gewebeschlauch sein kann, und einen ersten Endabschnitt 8 mit einer ersten Endöffnung 10 und einen zweiten Endabschnitt 9 mit einer zweiten Endöffnung 11 aufweist, ist auf der äußeren Umfangsfläche des Stents 2 angeordnet. Der Schlauch 3 ist bei der in Fig. 1A dargestellten Ausführungsform vollständig mit seinem ersten Endabschnitt 8 über den Stent 2 gezogen und schließt mit der ersten Endöffnung 6 des Stents 2 ab. Bei der in Fig. 1B dargestellten Ausführungsform ist zumindest ein Teil des ersten Endabschnitts 4 des Stents 2 freigelassen, d.h. nicht vom Schlauch 3 bedeckt. Der erste Endabschnitt 4 des Stents 2 ist dazu vorgesehen, in die Mündung einer Pulmonalvene eingeführt zu werden. Der Schlauch 3 steht mit seinem zweiten Endabschnitt 9 und der zweiten Endöffnung 11 über die zweite Endöffnung 7 des Stents 2 hinaus. Der Stent 2 weist hier zwei gegenüberliegende (s. Fig. 2) draht- oder bandförmige Stabilisierungselemente 12 auf, die ebenfalls über die zweite Endöffnung 7 des Stents 2 hinausstehen und den Schlauch 3 von innen stützen. Hier erstrecken sich die draht- oder bandförmige Stabilisierungselemente 12 im Wesentlichen bis zur zweiten Endöffnung 11 des Schlauchs 3. Die draht- oder bandförmige Stabilisierungselemente 12 sorgen dafür, dass die zweite Endöffnung 11 des flexiblen Schlauchs 3 im Neutralzustand geöffnet bleibt und nicht kollabiert.

Figur 2 zeigt vereinfachte räumliche Ansichten der in Figur 1 dargestellten Ausführungsformen der erfindungsgemäßen Klappenprothese 1. In Figur 2A ist die in Fig. 1A dargestellte Ausführungsform wiedergegeben, in Figur 2B die in Fig. 1B dargestellte Ausführungsform. Hier ist die Anordnung der Stabilisierungselemente 12 besser erkennbar. Pfeile geben die Richtung an, in die sich Schlauchwände beim Kollabieren des Schlauches 3 unter entsprechenden Druckverhältnissen bewegen.

Die beiden in den Figuren 1 und 2 dargestellten Ausführungsformen der erfindungsgemäßen Klappenprothese 1 unterscheiden sich lediglich darin, dass der erste Endabschnitt 4 des Stents 2 entweder vom Schlauch 3 bedeckt (A) oder freigehalten (B) ist. Die Ausführungsform mit freigehaltenem erstem Endabschnitt 4 führt gegenüber der hier mit dem Schlauch überzogenen Ausführungsform zu einer geringeren Verringerung des Querschnitts der Pulmonalvene 31, wenn der Stent 2 nur mit dem vom Schlauch 3 freigehaltenen Endabschnitt 4 in die Pulmonalvene 31 eingeführt wird.

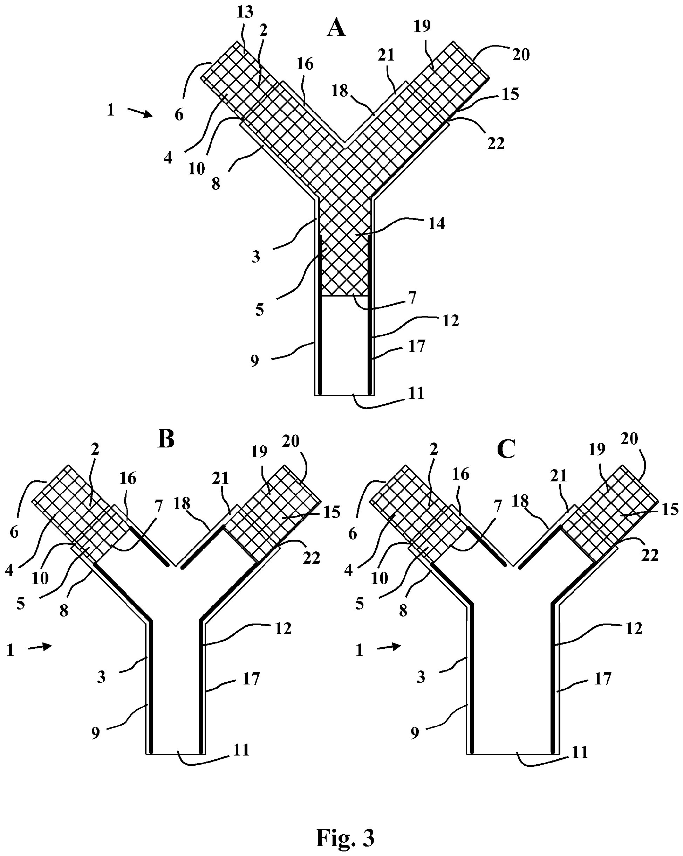

Figur 3 zeigt schematische Ansichten verschiedener verzweigter, Y-förmiger Ausführungsformen der erfindungsgemäßen Klappenprothese 1. Bei der in Figur 3A dargestellten Ausführungsform sind sowohl der Stent 2 als auch der Schlauch 3 Y-förmig verzweigt, während bei den in Figur 3B und Figur 3C dargestellten Ausführungsformen nur der Schlauch 3 Y-förmig verzweigt ist. Der Stent 3 der in Figur 3A dargestellten Y-förmig verzweigten Ausführungsform der erfindungsgemäßen Klappenprothese 1 ist in einen ersten, zweiten und dritten Schenkel 13, 14 und 15 untergliedert. Dementsprechend weist auch der auf dem Stent 3 aufgebrachte Schlauch 3 erste, zweite und dritte Schenkel 16, 17 und 18 auf. Der dritte Schenkel 15 des Stents 2 weist einen dritten Endabschnitt 19 des Stents 2 mit einer dritten Endöffnung 20 auf. Entsprechend weist auch der Schlauch einen dritten Endabschnitt 21 mit einer Endöffnung 22 auf. Der Schlauch 3 ist hier nicht über den gesamten Stent 2 vorgesehen, sondern lässt den ersten Endabschnitt 4 am ersten Schenkel 13 und den dritten Endabschnitt 19 am dritten Schenkel 15 des Stents 2 frei. Diese Endabschnitte 4, 19 sind zur Verankerung in benachbarten Pulmonalvenen 31 vorgesehen. Der Schlauch 3 steht mit seinem auf dem zweiten Schlauchschenkel 17 liegenden zweiten Endabschnitt 9 und seiner zweiten Endöffnung 11 über die zweite Endöffnung 7 des Stents 3 über, und wird in seinem freien Bereich von gegenüberliegenden mit dem Stent 3 fest verbundenen draht- oder bandförmigen Stabilisierungselementen 12 von innen gestützt.

Bei den in den Figuren 3B und 3C dargestellten Ausführungsformen der erfindungsgemäßen Klappenprothese 1 sind ebenfalls Y-förmig ausgestaltet. Allerdings ist hier lediglich der Schlauch 3 Y-förmig verzweigt. In den Endabschnitten 8, 21 der Schenkel 16, 18 des Schlauchs 3 befinden sich hohlzylinderförmige Stents 2, die hier nicht vollständig von dem Schlauch 3 überzogen sind, sondern mit ihren Endabschnitten 4, 19 freiliegen. Stabilisierungselemente 12 sind auch bei diesen Ausführungsformen vorgesehen, um den Schlauch von innen zu stützen und ein Kollabieren im Neutralzustand zu verhindern. Bei der in Figur 3C dargestellten Ausführungsform ist der Querschnitt des zweiten Schlauchschenkels 17 gegenüber dem Schlauchquerschnitt im Bereich der Schlauchschenkel 16, 18 erweitert, um im Einsatz eine Flussbegrenzung des aus zwei Pulmonalvenen 31 in dem Schlauch 3 vereinigten Blutstroms zu vermeiden.

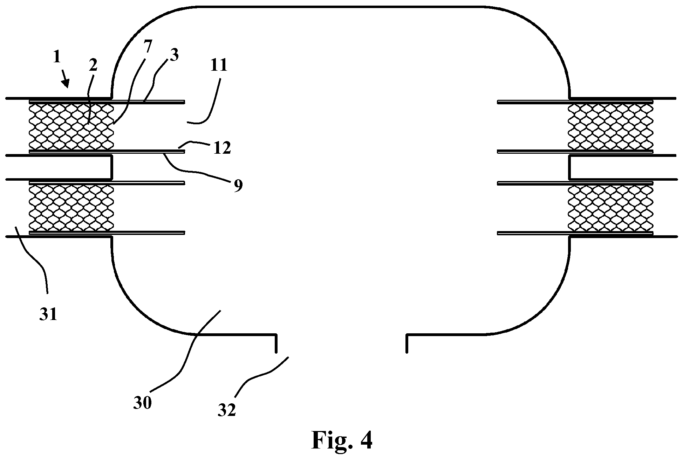

In Figur 4 ist stark schematisiert eine Einbausituation dargestellt. Dargestellt ist der linke Herzvorhof 30, in den die vier Pulmonalvenen 31 (obere und untere linke sowie obere und untere rechte Pulmonalvene) münden. Der Mitralklappenbereich als Übergang in den hier nicht dargestellten linken Ventrikel ist lediglich durch eine Öffnung 32 angedeutet. Dargestellt sind vier im Mündungsbereich der Pulmonalvenen 31 implantierte Ausführungsformen der erfindungsgemäßen Klappenprothese 1 gemäß den Figuren 1A und 2A. Der besseren Übersicht halber sind hier nicht sämtliche Bezugsziffern wiederholt. Die Stents 2 sind hier vollständig vom Schlauch 3 überzogen und fluchten mit ihren zweiten Endöffnungen 7 im Wesentlichen mit der Innenwand des Vorhofs 30. Die über die Endöffnungen 7 hinausstehenden zweiten Schlauchendabschnitte 9 ragen in den Vorhof 30 hinein und werden durch Stabilisierungselemente 12 so gestützt, dass ihre Endöffnungen 11 im Neutralzustand offen sind. Der Zufluss von Blut aus den Pulmonalvenen 31 in den Vorhof 30 wird durch die erfindungsgemäßen Klappenprothesen 1 nicht behindert. Bei Rückfluss von Blut aus dem Ventrikel in den Vorhof 30 aufgrund einer Mitralklappeninsuffizienz kollabieren die Schlauchendabschnitte 9 jedoch, so dass die Endöffnungen 11 verschlossen werden, wodurch ein Rückfluss von Blut in die Pulmonalvenen 31 verhindert wird.

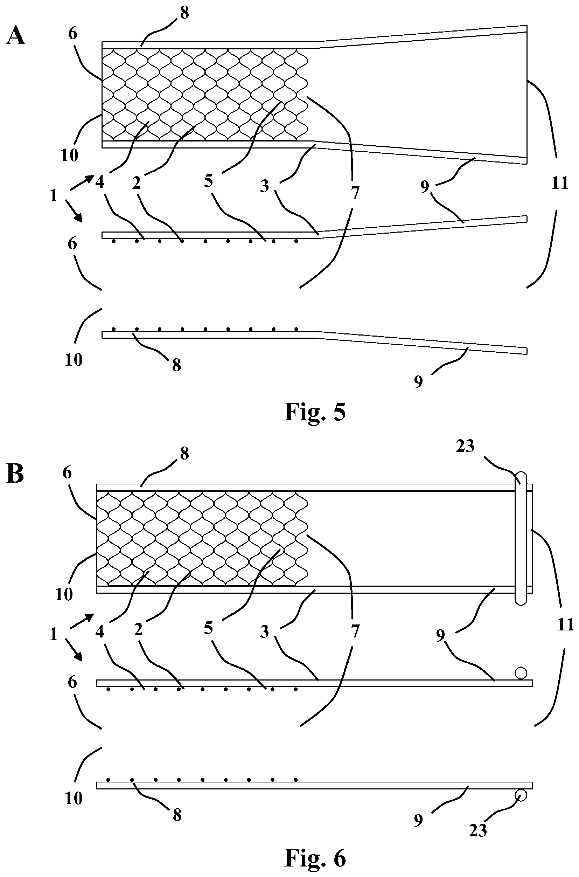

Die Figuren 5 und 6 zeigen seitliche Ansichten und Querschnitte durch zwei weitere Ausführungsformen der erfindungsgemäßen Klappenprothese 1. Bei der in Figur 5 dargestellten Ausführungsform erweitert sich der Querschnitt des Schlauches 3 konisch zu dessen zweiter Endöffnung 11 hin. Die zweite Endöffnung 11 ist somit weiter als die erste Endöffnung 10, wodurch ein etwaiges Zurückziehen des Schlauches 3 in den Stent 2, beispielsweise in Unterdrucksituationen, verhindert ist. Bei der in Figur 6 dargestellten Ausführungsform ist zu diesem Zweck ein ringförmiges Verstärkungselement 23 vorgesehen, das außen auf dem Schlauch 3 angeordet ist und im zweiten Endabschnitt 9 des Schlauches 3 eine Versteifung und Umfangserweiterung bewirkt, die ebenfalls ein Zurückziehen des Schlauches 3 in den Stent 2 verhindert.

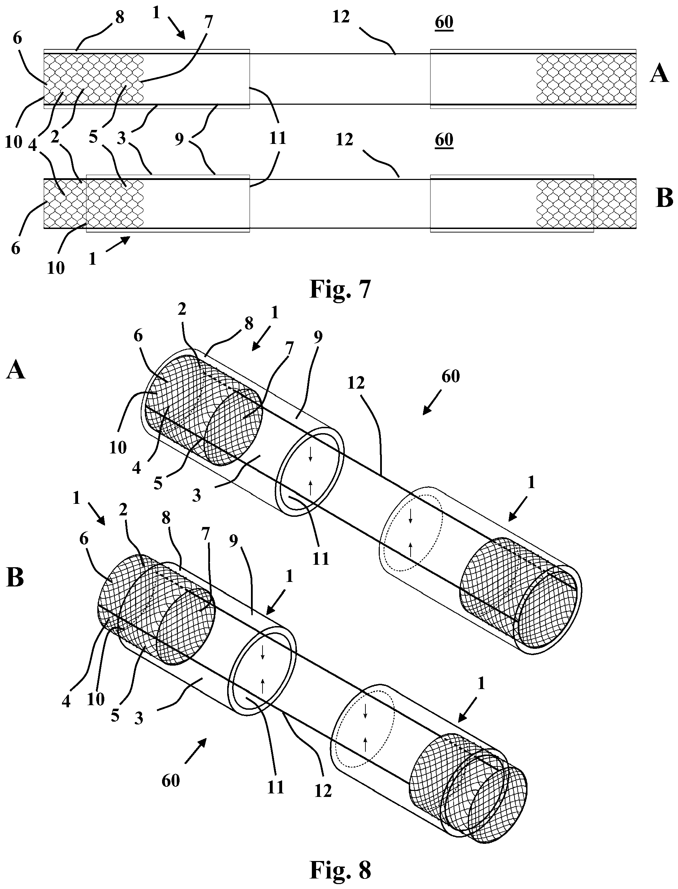

Die Figuren 7 und 8 zeigen zwei Ausführungsformen einer erfindungsgemäßen Klappenprothese 60, die besonders gut zum Einsatz in die Hohlvenen 41, 42 geeignet sind. Figur 7 zeigt die Ausführungsformen in einer seitlichen, Figur 8 in einer räumlichen Ansicht. Die in den Figuren 7A und 8A dargestellte Ausführungsform unterscheidet sich von der in den Figuren 7B und 8B dargestellten Ausführungsform darin, dass der erste Endabschnitt 4 des Stents 2 jeweils vom Schlauch 3 bedeckt ist, während er bei der in den Figuren 7B und 8B dargestellten Ausführungsform jeweils freigehalten ist.

Die in den Figuren 7 und 8 dargestellten Ausführungsformen einer erfindungsgemäßen Klappenprothese 60 bestehen jeweils aus zwei Klappenprothesen 1, wie sie in den Figuren 1 und 2 dargestellt sind, die mit den zweiten Endöffnungen 11 ihrer Schläuche 3 zueinander gerichtet angeordnet sind. Dabei sind die beiden Klappenprothesen 1 voneinander beabstandet, so dass ein freier Raum zwischen den Endöffnungen 11 ihrer Schläuche 3 verbleibt. Der freie Raum wird überbrückt durch zwei draht- oder bandförmige Stablisierungselemente 12, die miteinander verbunden bzw. einstückig miteinander ausgebildet sind. Auf diese Weise sind zwei erfindungsgemäße Klappenprothesen 1 zu einer Klappenprothese 60 vereint, die mit den jeweiligen ersten Endabschnitten 4 ihrer Stents 2 in die obere und untere Hohlvene 41, 42 implantiert werden kann.

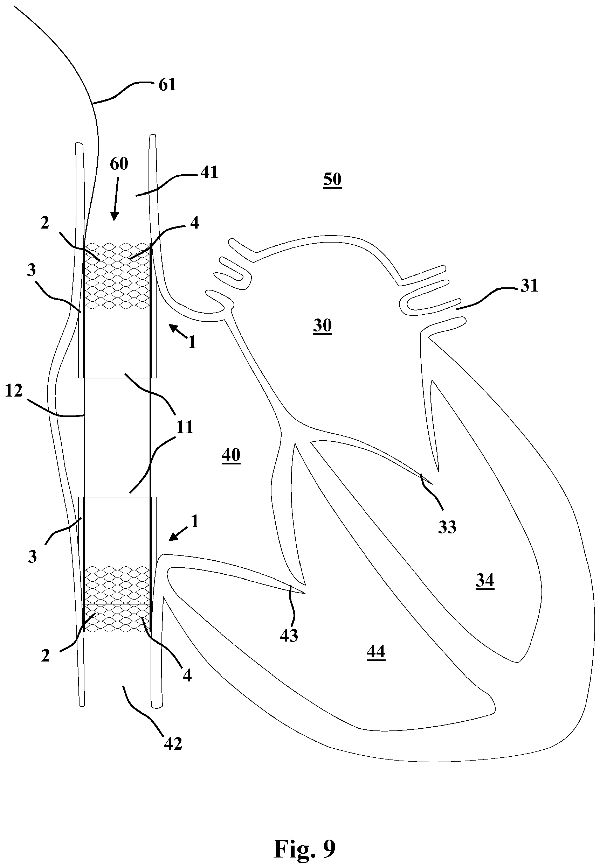

Figur 9 zeigt die in Figur 7B und Figur 8B dargestellte Ausführungsform der erfindungsgemäßen Klappenprothese 60 in implantierten Zustand. Dargestellt ist ein schematischer Querschnitt durch ein menschliches Herz mit dem linken Vorhof 30, der linken Herzkammer 34 und der dazwischen befindlichen Mitralklappe 33 sowie dem rechten Vorhof 40, der rechten Herzkammer 44 und der dazwischen liegenden Trikuspidalklappe 43. Pulmonalvenen 31 münden in den linken Vorhof 30, die obere Hohlvene 41 und die untere Hohlvene münden in den rechten Vorhof 40. Die erfindungsgemäße Klappenprothese 60 ist mit dem ersten Endabschnitt 4 des ersten Stents 2 in die oberen Hohlvene 41 und mit dem gegenüberliegenden ersten Endabschnitt 4 des zweiten Stents 2 in die untere Hohlvene 42 implantiert. Zur extravasalen Befestigung der erfindungsgemäßen Klappenprothese 60 ist hier ein drahtförmiges Befestigungselement (61) vorgesehen, welches beispielsweise über eine Implantationsvene (z.B. Vena subclavia) ausgeführt und z.B. mit chirurgischem Nahtmaterial extravasal fixiert werden kann.

Die zweiten Endöffnungen 11 der Klappenprothese 60 ragen frei in den rechten Vorhof 40, sind allerdings durch die draht- oder bandförmigen Stabilisierungselemente 12 stabilisiert. Aufgrund des Klappendesigns, bei dem die Klappenprothese 60 im Neutralzustand geöffnet ist, muss bei physiologischen Fluß aus den Hohlvenen 41, 42 in Richtung rechtem Vorhof 40 kein Widerstand überwunden werden. Der Flußwiderstand ist bei der hier beschriebenen erfindungsgemäßen Klappenprothese 60, die vorteilhaft bei Trikuspidalklappeninsuffizienz eingesetzt werden kann, nahezu vernachlässigbar. Bei einem unphysiologischen Fluss aus dem rechten Vorhof 40 in Richtung der Hohlvenen 41, 42 kommt es durch Unterdruck zu einem Kollaps der Klappenprothesen 1 und somit zu einem kompetenten Klappenschluss. Ein Rückstrom in die Hohlvenen 41, 42 und somit ein konsekutiver Blutrückstau in Leber und Nieren wird somit verhindert. Der Einsatz der erfindungsgemäßen Klappenprothese 60 kann völlig unabhängig von der Pathologie und den anatomischen Gegebenheiten der Trikuspidalklappe 43 erfolgen.

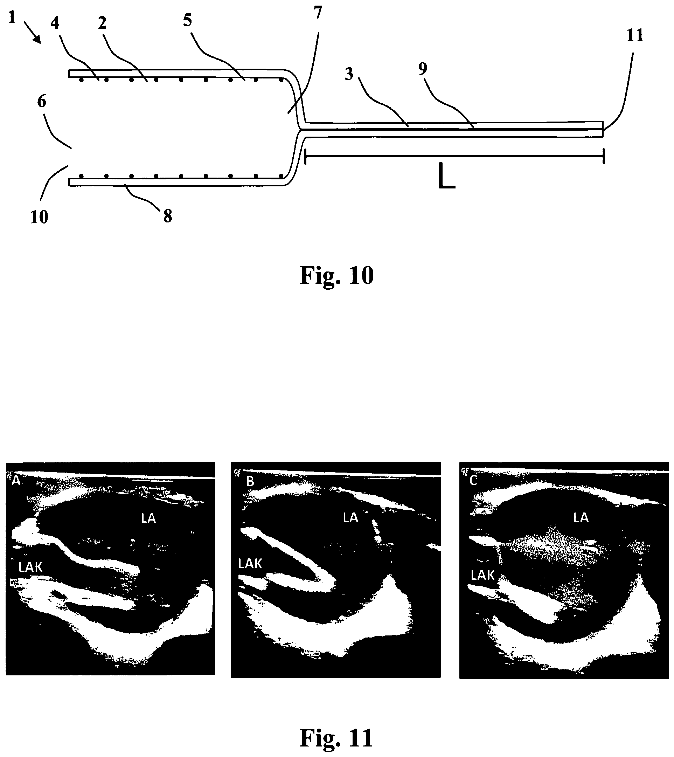

Figur 10 zeigt eine Schnittansicht einer Ausführungsform einer erfindungsgemäßen Klappenprothese 1, wie sie in Figur 1A dargestellt ist, in geschlossenem Zustand, d.h. beim Klappenschluss. Gegenüberliegende Anteile des über die zweite Endöffnung 7 des Stents 2 hinausstehenden Bereiches des Schlauches 3 liegen über die Koaptationslänge L eng aneinander, so dass ein kompetenter Schluss der Klappenprothese 1 erfogt.

### Ausführungsbeispiel

Mit Hilfe einer Modellapparatur wurde die Funktionalität der erfindungsgemäßen Klappenprothesen 1 bestätigt. Hierzu wurde ein Acrylglasbehälter mit zwei Öffnungen hergestellt. In eine der Öffnungen wurde eine Ausführungsform der erfindungsgemäßen Klappenprothese 1 gemäß Figur 1A, 2A, so montiert, dass diese mit dem Schlauchendabschnitt 9 frei in den Behälterinnenraum ragte. Auf dem ersten Endabschnitt des Stents war ein Schlauch zur Nachbildung einer Pulmonalvene montiert. Durch die zweite Öffnung wurde ein Schlauch in den Behälterinnenraum geführt, durch den zur Simulation einer Mitralklappeninsuffizienz farbige Flüssigkeit aus einem externen Behälter in den Behälterinnenraum geleitet werden konnte. Dadurch kommt es zusätzlich zu einer Druckerhöhung in dem Plexiglasbehälter. Hierdurch kommt es zum Kollaps der erfindungsgemäßen Klappenprothesen. Die erfindungsgemäße Klappenprothese 1 verhinderte zuverlässig durch Kollabieren des in den Behälter ragenden Schlauchteils einen Rückfluss von Flüssigkeit in den mit der Klappenprothese 1 verbundenen Schlauch.

Darüber hinaus wurde eine Klappenprothese 1, wie sie in den Figuren 1A und 2A dargestellt ist, nach Induktion einer hochgradigen Mitralklappeninsuffizienz linksatrial in Pulmonalvenen von Schweineherzen implantiert und mittels Echodardiographie und Kontrastmittel-Angiographie auf ihre Funktionalität untersucht.

Figur 11 zeigt eine Echodardiographie eines Schweineherzens mit linksatrial implantierter Klappenprothese (LAK). Figur A zeigt die erfindungsgemäße Klappenprothese 1 (LAK) in offenem, Figur B in sich schließendem und Figur C in vollständig geschlossenem Zustand. Eine Messung der Flussgeschwindigkeiten (nicht dargestellt) in der Klappenprothese 1 (LAK) und im linkenVorhof (LA) ergab erwartungsgemäß eine unidirektionale Flusskurve für die Klappenprothese 1 und einen Pendelfluss im linken Vorhof.

## Patentansprüche

1. Implantierbare Klappenprothese (1), insbesondere zur Verhinderung von Blutrückfluss aus einem Herzvorhof in eine in den Herzvorhof mündende Vene, umfassend
- einen allgemein röhrenförmigen Stent (2), der verzweigt oder unverzweigt sein kann und mindestens einen ersten Endabschnitt (4) mit einer ersten Endöffnung (6) und einen zweiten Endabschnitt (5) mit einer zweiten Endöffnung (7) aufweist, und
- einen flexiblen Schlauch (3), der verzweigt oder unverzweigt sein kann und mindestens einen ersten Endabschnitt (8) mit einer ersten Endöffnung (10) und einen zweiten Endabschnitt (9) mit einer durch Kollabieren des Schlauches (3) verschließbaren zweiten Endöffnung (11) aufweist, der zumindest in einem Teilbereich des zweiten Endabschnitts (9) des Stents (2) auf dessen äußerer Umfangsfläche so angeordnet ist, dass der Schlauch (3) mit seinem zweiten Endabschnitt (9) und der zweiten Endöffnung (11) über die zweite Endöffnung (7) des Stents (2) hinaussteht, wobei die zweite Endöffnung (11) des Schlauches (3) im drucklosen Zustand offen ist, **dadurch gekennzeichnet, dass** sich der Schlauch (3) zu seiner zweiten Endöffnung (11) hin im Querschnitt erweitert und/oder der Schlauch (3) in seinem zweiten Endabschnitt (9) ein außen am Schlauch befestigtes ringförmiges Verstärkungselement (23) aufweist, dessen Innenquerschnitt mindestens dem Innenquerschnitt der zweiten Endöffnung (7) des Stents (2) entspricht.

2. Implantierbare Klappenprothese (1) nach Anspruch 1, ferner umfassend
- mindestens ein am Stent (2) befestigtes oder mit dem Stent (2) integral ausgebildetes draht- oder bandförmiges Stabilisierungselement (12), wobei das Stabilisierungselement (12) über die zweite Endöffnung (7) des Stents (2) hinaussteht und den über die zweite Endöffnung (7) des Stents (2) hinausstehenden Teil des Schlauchs (3) so von innen stützt, dass die zweite Endöffnung (11) des Schlauches (3) in drucklosem Zustand offen ist und unter Druckbeaufschlagung nicht in die zweite Endöffnung (7) des Stents (2) gelangen kann.

3. Implantierbare Klappenprothese (1) nach Anspruch 2, wobei mindestens zwei einander gegenüberliegende den Schlauch (3) von innen stützende draht- oder bandförmige Stabilisierungselemente (12) vorhanden sind.

4. Implantierbare Klappenprothese (1) nach einem der Ansprüche 1 bis 3, wobei der Stent (2) und der Schlauch (3) unverzweigt und allgemein hohlzylindrisch ausgebildet sind.

5. Implantierbare Klappenprothese (1) nach einem der vorhergehenden Ansprüche, wobei der Schlauch (3) aus Körpergewebe, vorzugsweise aus menschlichem oder tierischem Körpergewebe, besteht.

6. Implantierbare Klappenprothese (1) nach einem der vorhergehenden Ansprüche, wobei der Stent (2) aus biokompatiblem Metall oder Kunststoff besteht.

7. Implantierbare Klappenprothese (1) nach einem der vorhergehenden Ansprüche, wobei der Stent (2) im Bereich seiner gesamten äußeren Umfangsfläche von dem Schlauch (3) überzogen ist.

8. Implantierbare Klappenprothese (1) nach einem der vorhergehenden Ansprüche, wobei der Schlauch (3) sich zu seiner zweiten Endöffnung (11) hin im Querschnitt erweitert und der Schlauch (3) in seinem zweiten Endabschnitt (9) ein außen am Schlauch befestigtes ringförmiges Verstärkungselement (23) aufweist, dessen Innenquerschnitt größer ist als der Innenquerschnitt der zweiten Endöffnung (7) des Stents (2).

9. Implantierbare Klappenprothese (1) nach einem der vorhergehenden Ansprüche, wobei die implantierbare Klappenprothese (1) so ausgestaltet ist, dass sich eine Koaptationslänge L von mindestens 1 cm, bevorzugt mindestens 1,2 cm, 1,3 cm oder 1,5 cm ergibt.

10. Implantierbare Klappenprothese (1) nach einem der Ansprüche 1 bis 3 oder 5 bis 9, wobei der Stent (2) und/oder der Schlauch (3) verzweigt und allgemein Y-förmig ausgebildet sind.

11. Implantierbare Klappenprothese (1) nach einem der vorhergehenden Ansprüche, wobei die implantierbare Klappenprothese (1) ein Befestigungselement (61) zur extravasalen Befestigung der implantierbare Klappenprothese (1) aufweist.

## Claims

1. An implantable valve prosthesis (1), in particular for preventing blood reflux from a cardiac atrium into a vein opening into the atrium, comprising
- a generally tubular stent (2) which may be branched or unbranched and which comprises at least a first end section (4) with a first end opening (6) and a second end section (5) with a second end opening (7), and
- a flexible tube (3) which may be branched or unbranched and which has at least a first end section (8) with a first end opening (10) and a second end section (9) with a second end opening (11) which can be closed by collapsing the tube (3) and which is disposed at least in a sub-region of the second end section (9) of the stent (2) on the outer peripheral surface thereof in a manner such that the second end section (9) of the tube (3) with its second end opening (11) protrudes beyond the second end opening (7) of the stent (2), wherein the second end opening (11) of the tube (3) is open in the unpressurized state, **characterized in that** the tube (3) widens in cross section towards its second end opening (11) and/or the tube (3) is provided in its second end section (9) with an annular reinforcing element (23) fastened to the outside of the tube, the internal cross section of the annular reinforcing element (23) having an internal cross section corresponding to at least the internal cross section of the second end opening (7) of the stent (2).

2. The implantable valve prosthesis (1) as claimed in claim 1, further comprising
- at least one wire- or ligature-shaped stabilization element (12) fastened to the stent (2) or integrally formed with the stent (2), wherein the stabilization element (12) protrudes beyond the second end opening (7) of the stent (2) and supports the portion of the tube (3) which protrudes beyond the second end opening (7) of the stent (2) from inside in a manner such that the second end opening (11) of the tube (3) is open in the unpressurized state and cannot gain ingress into the second end opening (7) of the stent (2) when pressure is applied.

3. The implantable valve prosthesis (1) as claimed in claim 2, wherein at least two wire- or ligature-shaped stabilization elements (12) which are opposite each other and which support the tube (3) from inside are present.

4. The implantable valve prosthesis (1) as claimed in one of claims 1 to 3, wherein the stent (2) and the tube (3) are unbranched and generally configured as a hollow cylinder.

5. The implantable valve prosthesis (1) as claimed in one of the preceding claims, wherein the tube (3) consists of body tissue, preferably of human or animal body tissue.

6. The implantable valve prosthesis (1) as claimed in one of the preceding claims, wherein the stent (2) consists of biocompatible metal or plastic.

7. The implantable valve prosthesis (1) as claimed in one of the preceding claims, wherein the stent (2) is covered by the tube (3) in the region of its entire outer peripheral surface.

8. The implantable valve prosthesis (1) as claimed in one of the preceding claims, wherein the tube (3) widens in cross section towards its second end opening (11) and wherein the second end section (9) of the tube (3) has an annular reinforcing element (23) fastened to the outside of the tube, the internal cross section of which element (23) being larger than the internal cross section of the second end opening (7) of the stent (2).

9. The implantable valve prosthesis (1) as claimed in one of the preceding claims, wherein the implantable valve prosthesis (1) is configured in a manner such that a coaptation length L of at least 1 cm, preferably at least 1.2 cm, 1.3 cm or 1.5 cm is produced.

10. The implantable valve prosthesis (1) as claimed in one of claims 1 to 3 or 5 to 9, wherein the stent (2) and/or the tube (3) are branched and generally Y-shaped in configuration.

11. The implantable valve prosthesis (1) as claimed in one of the preceding claims, wherein the implantable valve prosthesis (1) has a fastening element (61) for extravasal fastening of the implantable valve prosthesis (1).

## Revendications

1. Prothèse de valve implantable (1), en particulier pour empêcher un retour sanguin d'une oreillette du coeur dans une veine débouchant dans l'oreillette du coeur, comprenant
- un stent (2) généralement tubulaire qui peut être branché ou non branché et présente au moins un premier tronçon d'extrémité (4) avec une première ouverture d'extrémité (6) et un second tronçon d'extrémité (5) avec une seconde ouverture d'extrémité (7), et
- un tuyau flexible (3) qui peut être branché ou non branché et présente au moins un premier tronçon d'extrémité (8) avec une première ouverture d'extrémité (10) et un second tronçon d'extrémité (9) avec une seconde ouverture d'extrémité (11) pouvant être refermée par affaissement du tuyau flexible (3), qui est ainsi au moins disposé dans une zone partielle du second tronçon d'extrémité (9) sur la surface périphérique extérieure du stent (2) que le tuyau flexible (3) fait saillie par son second tronçon d'extrémité (9) et la seconde ouverture d'extrémité (11) au-dessus de la seconde ouverture d'extrémité (7) du stent (2), dans laquelle la seconde ouverture d'extrémité (11) du tuyau flexible (3) est ouverte dans l'état sans pression, **caractérisé en ce que** le tuyau flexible (3) s'élargit en section en direction de sa seconde ouverture d'extrémité (11) et/ou que le tuyau flexible (3) présente dans son second tronçon d'extrémité (9) un élément de renfort (23) annulaire fixé sur l'extérieur du tuyau flexible, dont la section intérieure correspond au moins à la section intérieure de la seconde ouverture d'extrémité (7) du stent (2).

2. Prothèse de valve implantable (1) selon la revendication 1, comprenant en outre
- au moins un élément de stabilisation (12) en forme de bande ou de fil fixé sur le stent (2) ou formé intégralement avec le stent (2), dans laquelle l'élément de stabilisation (12) fait saillie au-dessus de la seconde ouverture d'extrémité (7) du stent (2) et soutient de l'intérieur la partie du tuyau flexible (3) faisant saillie au-dessus de la seconde ouverture d'extrémité (7) du stent (2) de telle façon que la seconde ouverture d'extrémité (11) du tuyau flexible (3) est ouverte à l'état sans pression et ne peut pas parvenir dans la seconde ouverture d'extrémité (7) du stent (2) quand elle est sous pression.

3. Prothèse de valve implantable (1) selon la revendication 2, dans laquelle au moins deux éléments de stabilisation (12) en forme de bande ou de fil soutenant le tuyau flexible (3) de l'intérieur et opposés l'un à l'autre sont présents.

4. Prothèse de valve implantable (1) selon l'une des revendications 1 à 3, dans laquelle le stent (2) et le tuyau flexible (3) ne sont pas branchés et sont formés généralement en cylindre creux.

5. Prothèse de valve implantable (1) selon l'une des revendications précédentes, dans laquelle le tuyau flexible (3) est composé de tissu corporel, de préférence de tissu corporel humain ou animal.

6. Prothèse de valve implantable (1) selon l'une des revendications précédentes, dans laquelle le stent (2) est composé de métal ou de plastique biocompatible.

7. Prothèse de valve implantable (1) selon l'une des revendications précédentes, dans laquelle le stent (2) est recouvert par le tuyau flexible (3) au niveau de la totalité de sa surface périphérique extérieure.

8. Prothèse de valve implantable (1) selon l'une des revendications précédentes, dans laquelle le tuyau flexible (3) s'élargit en section en direction de sa seconde ouverture d'extrémité (11) et que le tuyau flexible (3) présente dans son second tronçon d'extrémité (9) un élément de renfort (23) annulaire fixé sur l'extérieur du tuyau flexible, dont la section intérieure est plus grande que la section intérieure de la seconde ouverture d'extrémité (7) du stent (2).

9. Prothèse de valve implantable (1) selon l'une des revendications précédentes, dans laquelle la prothèse de valve implantable (1) est ainsi conçue qu'il résulte une longueur de coaptation L d'au moins 1 cm, de préférence d'au moins 1,2 cm, 1,3 cm ou 1,5 cm.

10. Prothèse de valve implantable (1) selon l'une des revendications 1 à 3 ou 5 à 9, dans laquelle le stent (2) et/ou le tuyau flexible (3) sont branchés et forment généralement un Y.

11. Prothèse de valve implantable (1) selon l'une des revendications précédentes, dans laquelle la prothèse de valve implantable (1) présente un élément de fixation (61) pour la fixation extravasale de la prothèse de valve implantable (1).
